(19) Europäisches Patentamt
European Patent Office
Office européen des brevets

(11) Veröffentlichungsnummer: **0 280 145**
**A2**

# (12) EUROPÄISCHE PATENTANMELDUNG

(21) Anmeldenummer: **88102175.2**

(51) Int. Cl.⁴: **C07C 59/125**

(22) Anmeldetag: **15.02.88**

(30) Priorität: **25.02.87 DE 3706047**

(43) Veröffentlichungstag der Anmeldung:
**31.08.88 Patentblatt 88/35**

(84) Benannte Vertragsstaaten:
**AT BE CH DE ES FR GB GR IT LI LU NL SE**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Lange, Fritz, Dr.**
**Baderweg 82**
**D-4300 Essen(DE)**
Erfinder: **Gruber, Bert, Dr.**
**Marbacherstrasse 7**
**D-4000 Düsseldorf 13(DE)**
Erfinder: **Meffert, Alfred, Dr.**
**Marie-Curie-Strasse 10**
**D-4019 Monheim(DE)**
Erfinder: **Behler, Ansgar, Dr.**
**Wittekindstrasse 44**
**D-4250 Bottrop(DE)**

## (54) Erdalkalimetallsalze vicinal hydroxy, alkoxy-substituierter C16-C22-Fettsäuren, Verfahren zu ihrer Herstellung und ihre Verwendung als Alkoxylierungskatalysatoren.

(57) Erdalkalisalze vicinal hydroxy, alkoxy-substituierter $C_{16}$-$C_{22}$-Fettsäuren der allgemeinen Formel (I)

$$CH_3-(CH_2)_m-\overset{OH(OR)}{\overset{|}{CH}}-\underset{OR(OH)}{\underset{|}{CH}}-(CH_2)_n-COOM_{0,5}$$

in der R geradkettige oder verzweigte Alkyl-oder Alkenylgruppen oder Hydroxyalkylgruppen, die gegebenenfalls mit einer $C_{16}$-$C_{22}$-Fettsäure alkyliert sind, bedeuten, ergeben bei der Katalyse der Polyalkoxylierung von Verbindungen mit aktiven Wasserstoffatomen eine enge Homologenverteilung der Polyalkoxylierungsprodukte.

EP 0 280 145 A2

Niolenside mit eingeengter Homologenverteilung
Dodecanol +6EO

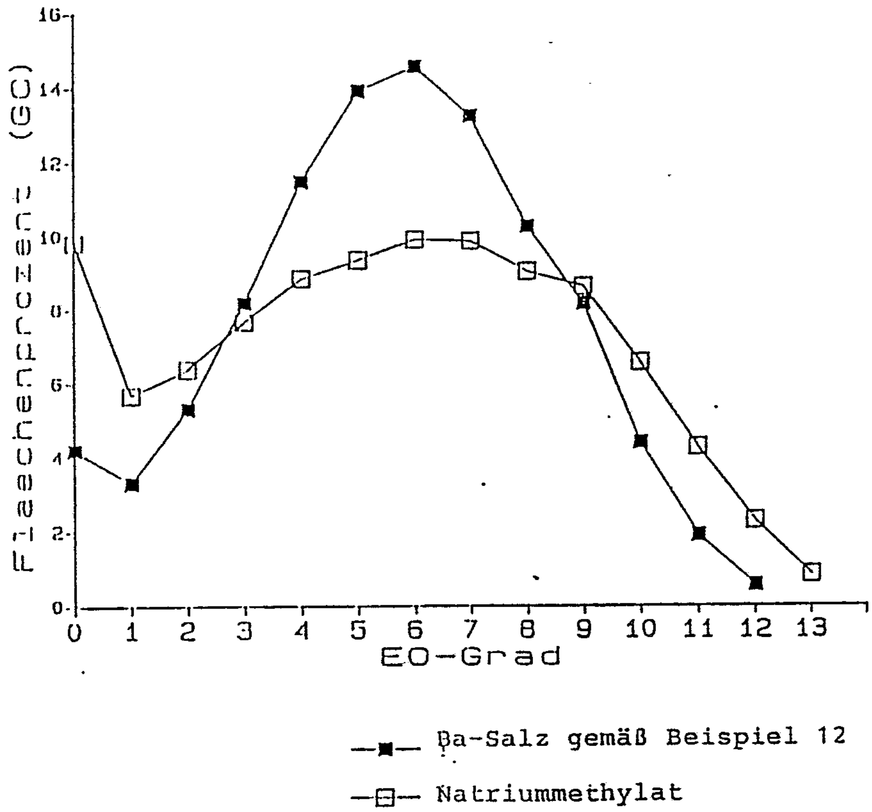

# Erdalkalimetallsalze vicinal hydroxy, alkoxy-substituierter $C_{16}$-$C_{22}$-Fettsäuren, Verfahren zu ihrer Herstellung und ihre Verwendung als Alkoxylierungskatalysatoren

Die Erfindung betrifft Erdalkalisalze vicinal hydroxy,alkoxy-substituierter $C_{16}$-$C_{22}$-Fettsäuren der allgemeinen Formel I

$$CH_3-(CH_2)_m-\overset{OH(OR)}{CH}-\underset{OR(OH)}{CH}-(CH_2)_n-COOM_{0,5} \qquad (I)$$

in der m und n jeweils ganze Zahlen bedeuten, die zusammen eine Summe von 12, 14, 16 oder 18 ergeben,
M ein Erdalkalimetall aus der von Mg, Ca, Sr und Ba gebildeten Gruppe ist, und
R aus einer der folgenden Gruppen ausgewählt ist:

a) geradkettige oder verzweigte Alkylgruppen mit 1 bis 22 Kohlenstoffatomen,

b) geradkettige oder verzweigte mono-ungesättigte Alkenylgruppen mit 3 bis 22 Kohlenstoffatomen,

c) Hydroxyalkylgruppen mit 2 bis 10 Kohlenstoffatomen und 1 bis 5 Hydroxylgruppen, und

d) Hydroxyalkylgruppen mit 2 bis 10 Kohlenstoffatomen und 1 bis 5 Hydroxylgruppen, bei denen eine Hydroxylgruppe mit dem Rest einer hydroxy-substituierten $C_{16}$-$C_{22}$-Carbonsäure der allgemeinen Formel (II)

$$CH_3-(CH_2)_m-\underset{OH}{CH}-\underset{(OH)}{CH}-(CH_2)_n-COOM_{0,5} \qquad (II)$$

in der m, n und M wie oben definiert sind, alkyliert ist.

Die Erfindung betrifft weiterhin Verfahren zur Herstellung der Erdalkalisalze der Formel (I) sowie die Verwendung derselben als Katalysatoren für die Ethoxylierung bzw. Propoxylierung von Verbindungen mit aktiven H-Atomen, z.B. von Fettalkoholen, Fettsäuren und Aminen.

Fettalkoholethoxylate und -propoxylate finden breite Verwendung als nicht-ionische Detergentien. Sie werden hergestellt, indem man Fettalkohole mit üblicherweise 10 bis 18 Kohlenstoffatomen mit Ethylenoxid oder Propylenoxid in Gegenwart von Katalysatoren umsetzt, wobei die Fettalkohole mit mehreren Molekülen Ethylenoxid oder Propylenoxid reagieren.

Als Katalysatoren für die vorgenannte Polyalkoxylierung sind hierfür u.a. die folgenden eingesetzt worden:
Calcium-und Strontiumhydroxide, -alkoxide und -phenoxide (EP-A 00 92 256),
Calciumalkoxide (EP-A 00 91 146),
Bariumhydroxid (EP-B 01 15 083),
basische Magnesiumverbindungen, z.B. Alkoxide (EP-A 00 82 569),
Magnesium-und Calciumfettsäuresalze (EP-A 0 85 167).

Gebräuchliche Polyalkoxylierungskatalysatoren sind weiterhin Kaliumhydroxid und Natriummethylat.

Die vorgenannten Katalysatoren weisen u.a. den Nachteil auf, daß sie schlecht in das Reaktionssystem einarbeitbar und/oder schwierig herstellbar sind.

Für Fettalkoholpolyalkoxylate ist eine enge Bandbreite des Polyalkoxylierungsgrades von besonderer Bedeutung, vgl. JAOCS, Vol. 63, 691-695 (1986), und HAPPI, 52-54 (1986). Die sogenannten "narrow-range"-Alkoxylate weisen demnach insbesondere die folgenden Vorteile auf:
- niedrige Fließpunkte
- höhere Rauchpunkte
- weniger Mole Alkoxid zum Erreichen der Wasserlöslichkeit
- weniger Hydrotope für das Einbringen in HDL's
- ein geringerer, durch Anwesenheit freier (nicht umgesetzter) Fettalkohole bedingter Geruch
- geringere Dunstbildung beim Sprühtrocknen.

Die Bandbreite bzw. Homologenverteilung der Fettalkoholpolyalkoxylate hängt wesentlich von der Art des verwendeten Katalysators ab. Als Maß für die Homologenverteilung gilt der sogenannte Q-Wert gemäß

der Beziehung

$Q = \bar{n} \bullet p^2$

in der $\bar{n}$ die durchschnittliche Adduktzahl (mittlerer Ethoxylierungsgrad) und p den Prozentsatz des Adduktes mit bestimmtem EO-Grad, das überwiegend gebildet wird, bedeuten. Ein großer Q-Wert bedeutet somit eine enge Bandbreite der Homologenverteilung.

Es wurde nun gefunden, daß man ebenfalls zu polyethoxylierten bzw. polypropoxylierten Fettalkoholen mit hohem Q-Wert kommt, wenn man die Erdalkalisalze der Erfindung als Ethoxylierungs-bzw. Propoxylierungskatalysatoren einsetzt. Gegenüber den vorbekannten Katalysatoren weisen die Erdalkalisalze der Erfindung den Vorteil auf, daß sie kurze Reaktionszeiten ermöglichen, bei Umgebungstemperatur flüssig oder wachsartig und somit besser dosierbar sind und schließlich im Reaktionssystem, anders als z.B. Erdalkalisalze gemäß dem Stand der Technik, löslich sind. Die gleichen Vorteile werden erhalten, wenn man die Erdalkalisalze der Erfindung als Katalysatoren zur Ethoxylierung bzw. Propoxylierung von anderen Verbindungen mit aktiven H-Atomen, z.B. von Fettsäuren und Aminen einsetzt.

Zwar sind aus der EP-A 00 22 236 Lithiumseifen bekannt, die zum Teil formal unter die allgemeine Formel (I) (M = Li) fallen, wobei der Rest R von geradkettigen oder verzweigten, gegebenenfalls ungesättigten Monoalkoholen mit bis zu 18 Kohlenstoffatomen gebildet ist; diese Lithiumseifen werden jedoch als Verdickungsmittel für Schmierfette eingesetzt und eignen sich nicht als Katalysatoren für die narrow-range-Polyalkoxylierung.

Das Grundgerüst der Erdalkalisalze der Erfindung leitet sich von mono-ungesättigten Fettsäuren mit 16, 18, 20 oder 22 Kohlenstoffatomen ab, insbesondere von Palmitoleinsäure (9-Hexadecensäure), Petroselinsäure (6-Octadecensäure), Ölsäure (9-Octadecensäure), Vaccensäure (11-Octadecensäure), 11-Octadecensäure, Gadoleinsäure (9-Eicosensäure), 11-Eicosensäure, Cetoleinsäure (11-Docosensäure) und Erucasäure (13-Docosensäure). Üblicherweise werden diese Fettsäuren aus Naturstoffen wie Talg und/oder pflanzlichen oder tierischen einschließlich seetierischen Ölen in Form von technischen Gemischen enthaltene kleinere Mengen an gesättigten oder mehrfach ungesättigten Fettsäuren oder Fettsäuren mit kleinerer oder größerer Kohlenstoffzahl oder hydroxysubstituierten Fettsäuren wie Ricinolsäure stören nicht. Selbstverständlich lassen sich auch reine $C_{16}$-$C_{22}$-Fettsäuren einsetzen; dies ist aus Kostengründen in den meisten Fällen jedoch unzweckmäßig.

Die vorgenannten Fettsäuren lassen sich in Form ihrer Ester mit $C_1$-$C_4$-Alkoholen in üblicher Weise epoxidieren, z.B. gemäß US-PS 2 485 160 sowie Chemical Week, April 1963, Seiten 55-60 und 64.

Die den Erdalkalisalzen der Erfindung zugrunde liegenden vicinal hydroxy, alkoxy-substituierten $C_{16}$-$C_{22}$-Fettsäuren sind Ringöffnungsprodukte der vorgenannten Epoxide mit Hydroxyverbindungen der Formel ROH, wobei sich durch die Struktur bedingt nicht angeben läßt, an welchem Kohlenstoffatom der Oxirangruppierung der Alkoxyrest angreift. Die Gruppe R kann dabei eine geradkettige oder verzweigte Alkylgruppe mit 1 bis 22 Kohlenstoffatomen sein, insbesondere eine Methyl-, Ethyl-, Propyl-, Butyl-, Pentyl-, Hexyl-, Heptyl-oder Octylgruppe, weiterhin eine von Fettalkoholen mit 10 bis 22 Kohlenstoffatomen abgeleitete Alkylgruppe. Weiterhin kann R auch eine von den entsprechenden geradkettigen oder verzweigten mono-ungesättigten Alkanolen mit 3 bis 22 Kohlenstoffatomen abgeleitete Alkylgruppe sein. Insoweit bevorzugt sind Methyl-, Butyl-, und Octyl-gruppen sowie von $C_{16}$/$C_{18}$-Schnitten von Fettalkoholen, hergestellt aus Naturprodukten, abgeleitete Alkylgruppen.

Gemäß einer weiteren Ausführungsform der Erfindung kann die Gruppe R eine Hydroxyalkylgruppe mit 2 bis 10 Kohlenstoffatomen und 1 bis 5 Hydroxylgruppen sein, insbesondere abgeleitet aus der von Ethylenglykol, Propylenglykol, Butylenglykol, Glycerin, Diglycerin, Triglycerin, Trimethylolpropan, Di-trimethylolpropan, Pentaerythrit, Dipentaerythrit, Neopentylglykol, Mannit und Sorbit gebildeten Gruppe von mehrwertigen Alkoholen. Bei derartigen Ringöffnungsprodukten weist der Rest R somit freie Hydroxylgruppen auf. Bei geeigneter Reaktionsführung (Molverhältnis von Epoxid zu mehrwertigem Alkohol von mindestens 2 : 1) kann ein Mol des mehrwertigen Alkohols mit 2 Mol des epoxidierten $C_{16}$-$C_{22}$-Carbonsäureesters reagieren. Man erhält dann nach Verseifung und Umsetzung zu den Erdalkalisalzen Verbindungen der allgemeinen Formel (I), in der R der Rest eines mehrfunktionellen Alkohols ist, welcher seinerseits mit einem Rest einer hydroxy-substituierten $C_{16}$-$C_{22}$-Carbonsäure der allgemeinen Formel (II) alkyliert ist, wobei sich wiederum nicht angeben läßt, an welchem C-Atom der Carbonsäure die Ringöffnung des Oxiranringes erfolgt.

Als Erdalkalisalze der Erfindung sind die Calcium-oder Bariumsalze bevorzugt, die im übrigen auch in Form eines Gemisches derselben eingesetzt werden können.

Die Erfindung ist weiterhin auf ein Verfahren zur Herstellung der Erdalkalisalze der Erfindung gerichtet, das von einem Epoxid der allgemeinen Formel (III)

$$CH_3-(CH_2)_m-\underset{\diagdown O \diagup}{CH-CH}-(CH_2)_n-COOR^1 \qquad (III)$$

ausgeht, wobei m und n wie oben definiert sind und $R^1$ eine Alkylgruppe mit 1 bis 4 Kohlenstoffatomen bedeutet. Das Epoxid der allgemeinen Formel (III) wird in Gegenwart saurer oder alkalischer Katalysatoren mit der Hydroxyverbindung der allgemeinen Formel R-OH (IV), in der R wie oben definiert ist, einer Ringöffnungsreaktion unterworfen. Diese Reaktion ist grundsätzlich bekannt, vgl. Bull. Jap. Petrol. Ind. 7, 25-30 (1965), insbesondere Seiten 26 und 27. Der erhaltene, vicinal hydroxy, alkoxy-substituierte Ester der allgemeinen Formel (V)

$$CH_3-(CH_2)_m-\overset{OH(OR)}{\overset{|}{C}H}-\underset{OR(OH)}{\underset{|}{C}H}-(CH_2)_n-COOR^1 \qquad (V)$$

in der m, n, R und $R^1$ wie oben definiert sind, wird anschließend verseift und die erhaltene Carbonsäure wird in die Erdalkalisalze (I) überführt.

Die Erfindung wird im folgenden anhand bevorzugter Ausführungsbeispiele näher erläutert.

Typische Vertreter handelsüblicher epoxidierter Ölsäuremethylester, die bevorzugt in dem Verfahren der Erfindung eingesetzt werden können, weisen die in Tabelle I wiedergegebene Kettenlängenverteilung auf.

## Tabelle I

Kettenlängenverteilung in Gew.-% eines typischen Ölsäuremethylesters

| | | |
|---|---|---|
| 0 - 2 % | $C_{12}$ | |
| 2 - 5 % | $C_{14}$ | |
| 5 - 7 % | $C_{16}$ | |
| 5 - 7 % | $C_{16}'$ | (einfach ungesättigt) |
| 1 - 3 % | $C_{18}$ | |
| 70 - 75 % | C18' | (einfach ungesättigt) |
| 8 - 12 % | $C_{18}''$ | (zweifach ungesättigt) |
| 0 - 1 % | $C_{18}'''$ | (dreifach ungesättigt) |
| 0 - 4 % | höhere | |

Epoxidzahl: 4,55 Gew.-% Epoxidsauerstoff

## Tabelle II

Kettenlängenverteilung der Hauptbestandteile des speziell eingesetzten Ölsäuremethylesters (gaschromatografische Bestimmung)

| Me-Ester | Flächen-% |
|---|---|
| $C_{12}$ | 0,63 |
| $C_{14}$ | 2,74 |
| $C_{15}$ | 0,21 |
| $C_{16}$ | 4,21 |
| $C_{16}'$ | 5,01 |
| $C_{18}$ | 1,20 |
| $C_{18}'$ | 67,96 |
| $C_{18}''$ | 7,54 |
| $C_{18}'''$ | 0,72 |
| $C_{20}'$ | 1,01 |

In den Ausführungsbeispielen wurde speziell ein epoxidierter Ölsäuremethylester eingesetzt, der aus einem Ölsäuremethylester mit einer Kettenlängenverteilung gemäß Tabelle II hergestellt wurde.

Beispiele 1 bis 12

A. Allgemeine Arbeitsvorschriften.

Alkohol und Schwefelsäure werden im Reaktionsgefäß vorgelegt und auf die jeweilige Reaktionstemperatur gebracht. Danach läßt man den epoxidierten Fettsäureester innerhalb von einer Stunde zulaufen und innerhalb einer weiteren Stunde abreagieren. Danach kühlt man auf ca. 50°C und neutralisiert mit Natriummethylat.

Eingesetzte Mengen, Reaktionstemperaturen und Kennzahlen der erhaltenen hydroxy, alkoxy-Stearinsäureester sind in Tabelle III zusammengefaßt.

B. Verseifung der in Schritt A erhaltenen vicinal hydroxy, alkoxy-substituierten Stearinsäure.

Die Produkte der Beispiele 1 bis 4 gemäß Tabelle III werden bei 40°C mit einem Überschuß wässriger Natriumhydroxidlösung versetzt und 3 Stunden bei 90 bis 100°C belassen. Nach vollendeter Verseifung wird auf 70°C abgekühlt und mit konzentrierter Salzsäure auf einen pH von ca. 2 angesäuert. Die wässrige Phase wird heiß abgetrennt und das Produkt zweimal mit 60°C warmen Wasser gewaschen sowie anschließend im Vakuum getrocknet.

Einsatzmengen und Kennzahlen der erhaltenen Produkte sind in Tabelle IV zusammengefaßt.

Tabelle III

Ringöffnung des epoxydierten Ölsäuremethylesters mit Hydroxyverbindungen

| Beispiel | Epoxyfettsäuremethylester (g) | $H_2SO_4$ (g) | Methanol (g) | Ethylenglykol (g) | Trimethylolpropan (g) | Glycerin (g) | Reaktionstemperatur (°C) | OH-Zahl (mg KOH/g) | Verseifungszahl |
|---|---|---|---|---|---|---|---|---|---|
| 1 | 3868 | 8,8 | 2112 | | | | 65 | 146,5 | 170,8 |
| 2 | 3868 | 2,2 | | 2046 | | | 80 | 252,1 | 158,7 |
| 3 | 3868 | 1,65 | | | 2952 | | 90 | 343,8 | 130,4 |
| 4 | 3868 | 3,3 | | | | 2024 | 90 | 299,0 | 153,6 |

Tabelle IV

Verseifung der vicinal hydroxy, alkoxy-substituierten Stearinsäureester gemäß Tabelle III

| Beispiel | Hydroxy, alkoxy-stearinsäureester (g) | NaOH (g) | $H_2O$ (g) | OH-Zahl (mg KOH/g) | Säurezahl (mg KOH/g) |
|---|---|---|---|---|---|
| 1 | 2628 | 480 | 1200 | 145,3 | 170,9 |
| 2 | 2430 | 420 | 1400 | 250,6 | 158,0 |
| 3 | 2602 | 360 | 1400 | 323,5 | 130,2 |
| 4 | 2192 | 360 | 1400 | 288,1 | 147,5 |

C. Herstellung der Barium-bzw. Calciumsalze der vicinal hydroxy, alkoxy-substituierten Stearinsäuren gemäß Tabelle IV.

Die Stearinsäuren gemäß den Beispielen 1 bis 4 der Tabelle IV werden in einem Gemisch von Wasser und Isopropanol (1 : 1) gelöst bzw. aufgeschlämmt und bei 90°C mit äquimolaren Mengen Barium-bzw. Calciumacetat (Menge berechnet über die Säurezahl) umgesetzt. Die frei werdende Essigsäure wird mit dem Lösemittel abdestilliert.

Die Erdalkalisalze gemäß den Beispielen 1 bis 4 sind in der folgenden Tabelle V zusammen mit weiteren hergestellten Erdalkalisalzen der Erfindung zusammengefaßt.

D. Ethoxylierung mit Erdalkalisalzen vicinal hydroxy, alkoxy-substituierter Stearinsäuren als Katalysator.

Der Katalysator wird in der Substanz mit aktivem Wasserstoff gelöst, wobei die Katalysatorkonzentration vorzugsweise 0,5 bis 1,5 Gew.-%, bezogen auf das Endprodukt, beträgt. Die Lösung wird in einen für die Alkoxylierung geeigneten Autoklaven überführt. Man spült mit Stickstoff und evaluiert 30 Minuten lang bei einer Temperatur von 100°C. Anschließend wird die Temperatur auf 180°C gesteigert und bei einem Druck von 5 bar wird die gewünschte Menge Ethylenoxid aufgedrückt. Nach Beendigung der Reaktion läßt man 30 Minuten nachreagieren.

Die nach dieser Arbeitsvorschrift mit den Katalysatoren der Erfindung bei der Ethoxylierung eines handelsüblichen Laurylalkohols (Lorol® $C_{12}$) erhaltenen Ergebnisse sind in Tabelle VI zusammengefaßt. Es bedeuten:

Q = der obengenannte Q-Wert

$n_{max}$ = Ethoxylierungsgrad des Homologen, das im Produkt mengenmäßig am häufigsten auftritt.

OH-Zahl, ist/soll = OH-Zahlen der als Endprodukt erhaltenen polyethoxylierten Laurylalkohole

Kat-% = Katalysatorkonzentration, bezogen auf das Endprodukt

FFA = Gehalt des Endproduktes an freien Fettalkoholen in Flächen-% (GC-Analyse)

t = Reaktionszeit der Polyethoxylierung.

In Tabelle VII sind die Ergebnisse von Vergleichsversuchen mit bekannten Katalysatoren zusammengefaßt. Es wird ebenfalls ein handelsüblicher Laurylalkohol polyethoxyliert. Der Vergleich zeigt, daß die Verbindungen der Erfindung bessere Homologenverteilungen (größere Q-Werte) als Kaliumhydroxid, Natriummethylat und Calciumhydroxid ergeben. Sie liefern weiterhin ähnlich gute Ergebnisse wie Bariumhydroxid, Calciumoleat und Calciumstearat, sind im Gegensatz zu diesen jedoch in dem Reaktionsgemisch löslich. Calciummethylat, das ebenfalls ähnlich gute Ergebnisse liefert, ist wesentlich schwerer herzustellen und nur unter Schwierigkeiten handhabbar.

Schließlich wird in Figur 1 die mit der Verbindung des Beispiels 12 bei der Polyethoxylierung von Laurylalkohol erreichte enge Homologenverteilung der Polyethoxylierungsprodukte im Vergleich zu der mit Natriummethylat erzielten grafisch dargestellt.

Tabelle V

Hergestellte vicinal hydroxy, alkoxy-substituierte Erdalkalistearate der Erfindung.

$$CH_3-(CH_2)_7-\overset{OH(OR)}{CH}-\underset{OR(OH)}{CH}-(CH_2)_7-COOM_{0,5}$$

| Beispiel | R | M |
|---|---|---|
| 1 | Methyl | Ca |
| 2 | Hydroxyethyl | Ca/Ba |
| 3 | Trimethylolpropyl | Ca |
| 4 | 2,3-Dihydroxypropyl | Ca |
| 5 | $C_8$-Alkyl | Ca |
| 6 | $C_8$-Alkyl | Ba |
| 7 | $C_{16}/C_{18}$'-Alkyl | Ca |
| 8 | $C_{16}/C_{18}$'-Alkyl | Ba |
| 9 | 4-Hydroxybutyl | Ca |
| 10 | 4-Hydroxybutyl | Ba |
| 11 | Trimethylolpropyl | Ba |
| 12 | 2,3-Dihydroxypropyl | Ba |

## Tabelle VI

Ethoxylierung eines handelsüblichen Laurylalkohols (Lorol[R] $C_{12}$) unter Verwendung der Verbindungen der Erfindung als Katalysator

| Kat. aus Beispiel | Kat-% | Rkt.Zeit (h) | Q | $n_{max}$ | OH-Zahl ist | OH-Zahl soll | FFA (%) |
|---|---|---|---|---|---|---|---|
| 1 | 1 | 7 | 1244 | 6 | 129,8 | 125,7 | 6,2 |
| 3 | 0,5 | 10,5 | 1161 | 6 | 137,9 | 130,0 | 4,2 |
| 4 | 0,5 | 7 | 1144 | 6 | 126,5 | 125,2 | 4,5 |
| 5 | 5 | 6 | 985 | 6 | 134,4 | 134,1 | 6,9 |
| 6 | 0,5 | 1,5 | 1242 | 6 | 125,5 | 123,7 | 4,2 |
| 6 | 0,1 | 8,5 | 1297 | 6 | 126,9 | 125,7 | 8,5 |
| 7 | 5 | 4 | 1217 | 5 | 127,9 | 119,8 | 7 |
| 8 | 0,5 | 4 | 1123 | 6 | 130,3 | 124,7 | 6 |
| 9 | 0,5 | 14,5 | 1210 | 6 | 125,4 | 125,7 | 3,3 |
| 10 | 0,5 | 4 | 1061 | 5 | 127,9 | 123,7 | 5,2 |
| 11 | 0,5 | 3 | 1081 | 5 | 146 | 131,2 | 6 |
| 12 | 0,5 | 1,5 | 1286 | 6 | 128,3 | 128,4 | 4,2 |

Tabelle VII

Ethoxylierung eines handelsüblichen Laurylalkohols (Lorol[R] $C_{12}$) unter Verwendung bekannter Katalysatoren

| Katalysator. | Kat-% | Rkt. Zeit (h) | Q | $n_{max}$ | OH-Zahl ist | soll | FFA (%) |
|---|---|---|---|---|---|---|---|
| KOH | 0,5 | 3,5 | 611 | 6 | 125,1 | 125,2 | 7,5 |
| $NaOCH_3$ | 0,5 | 4,0 | 595 | 6 | 128,8 | 126,8 | 9,9 |
| $Ca(OH)_2$ | 0,5 | 11,0 | 695 | 6 | 136,8 | 126,3 | 9,4 |
| $Ba(OH)_2$ | 1,0 | 4,0 | 1225 | 6 | 129,3 | 128,4 | 3,8 |
| Ca-Oleat | 0,5 | 10,5 | 1272 | 6 | 126,7 | 128,4 | 5,1 |
| Ca-Stearat | 1,0 | 7,5 | 1251 | 6 | 132,8 | 129,5 | 5,0 |
| Ca-Ethylat | 0,5 | 8,0 | 1316 | 6 | 130,2 | 125,7 | 4,5 |

**Ansprüche**

1. Erdalkalisalze vicinal hydroxy,alkoxy-substituierter $C_{16}$-$C_{22}$-Fettsäuren der allgemeinen Formel I

$$CH_3-(CH_2)_m-\underset{\underset{OR(OH)}{|}}{\overset{\overset{OH(OR)}{|}}{CH}}-CH-(CH_2)_n-COOM_{0,5} \qquad (I)$$

in der m und n jeweils ganze Zahlen bedeuten, die zusammen eine Summe von 12, 14, 16 oder 18 ergeben,

M ein Erdalkalimetall aus der von Mg, Ca, Sr, und Ba gebildeten Gruppe ist, und
R aus einer der folgenden Gruppen ausgewählt ist:
a) geradkettige oder verzweigte Alkylgruppen mit 1 bis 22 Kohlenstoffatomen,
b) geradkettige oder verzweigte mono-ungesättigte Alkenylgruppen mit 3 bis 22 Kohlenstoffatomen,
c) Hydroxyalkylgruppen mit 2 bis 10 Kohlenstoffatomen und 1 bis 5 Hydroxylgruppen, und
d) Hydroxyalkylgruppen mit 2 bis 10 Kohlenstoffatomen und 1 bis 5 Hydroxylgruppen, bei denen eine Hydroxylgruppe mit dem Rest einer hydroxy-substituierten $C_{16}$-$C_{22}$-Carbonsäure der allgemeinen Formel (II)

$$CH_3-(CH_2)_m-\underset{OH}{CH}-\underset{(OH)}{CH}-(CH_2)_n-COOM_{0,5} \qquad (II)$$

in der m, n und M wie oben definiert sind,
alkyliert ist.

2. Erdalkalisalze nach Anspruch 1, dadurch gekennzeichnet, daß R eine aus der von Methyl, Butyl und Octyl gebildeten Gruppe ausgewählte Alkylgruppe ist.

3. Erdalkalisalze nach Anspruch 1, dadurch gekennzeichnet, daß R eine von natürlichen $C_{16}/C_{22}$-Fettalkoholen abgeleitete Alkyl-bzw. Alkenylgruppe ist.

4. Erdalkalisalze nach Anspruch 1, dadurch gekennzeichnet, daß R eine aus der von Ethylenglykol, Propylenglykol, Butylenglykol, Glycerin, Diglycerin, Triglycerin, Trimethylolpropan, Di-trimethylolpropan, Pentaerythrit, Dipentaerythrit, Neopentylglykol, Mannit und Sorbit gebildeten Gruppe abgeleitete Hydroxyalkylgruppe ist.

5. Erdalkalisalze nach einem der Ansprüche 1 bis 4, dadurch gekennzeichnet, daß M Ca oder Ba bedeutet.

6. Verfahren zur Herstellung der Erdalkalisalze nach einem der Ansprüche 1 bis 5, dadurch gekennzeichnet, daß man ein Epoxid der allgemeinen Formel (III)

$$CH_3-(CH_2)_m-\underbrace{CH-CH}_{O}-(CH_2)_n-COOR^1 \qquad (III)$$

in der m und n wie oben definiert sind und $R^1$ eine Alkylgruppe mit 1 bis 4 Kohlenstoffatomen bedeutet, in Gegenwart von sauren oder alkalischen Katalysatoren mit einer Hydroxyverbindung der allgemeinen Formel (IV)

R-OH (IV)

in der R wie oben definiert ist,
umsetzt und den erhaltenen, vicinal hydroxy,alkoxy-substituierten Ester der allgemeinen Formel (V)

$$CH_3-(CH_2)_m-\overset{OH(OR)}{CH}-\underset{OR(OH)}{CH}-(CH_2)_n-COOR^1 \qquad (V)$$

in der m, n, R und $R^1$ wie oben definiert sind, verseift, sowie die erhaltene Carbonsäure in die Erdalkalisalze (I) überführt.

7. Verfahren nach Anspruch 6, dadurch gekennzeichnet, daß man als Epoxid der allgemeinen Formel (III) ein epoxidiertes Gemisch technischer $C_{16}$-$C_{22}$-Fettsäuren verwendet.

8. Verwendung der Erdalkalisalze nach einem der Ansprüche 1 bis 7 als Katalysatoren in der Ethoxylierung und Propoxylierung von Fettalkoholen, Fettsäuren und Fettaminen.

Fig. 1

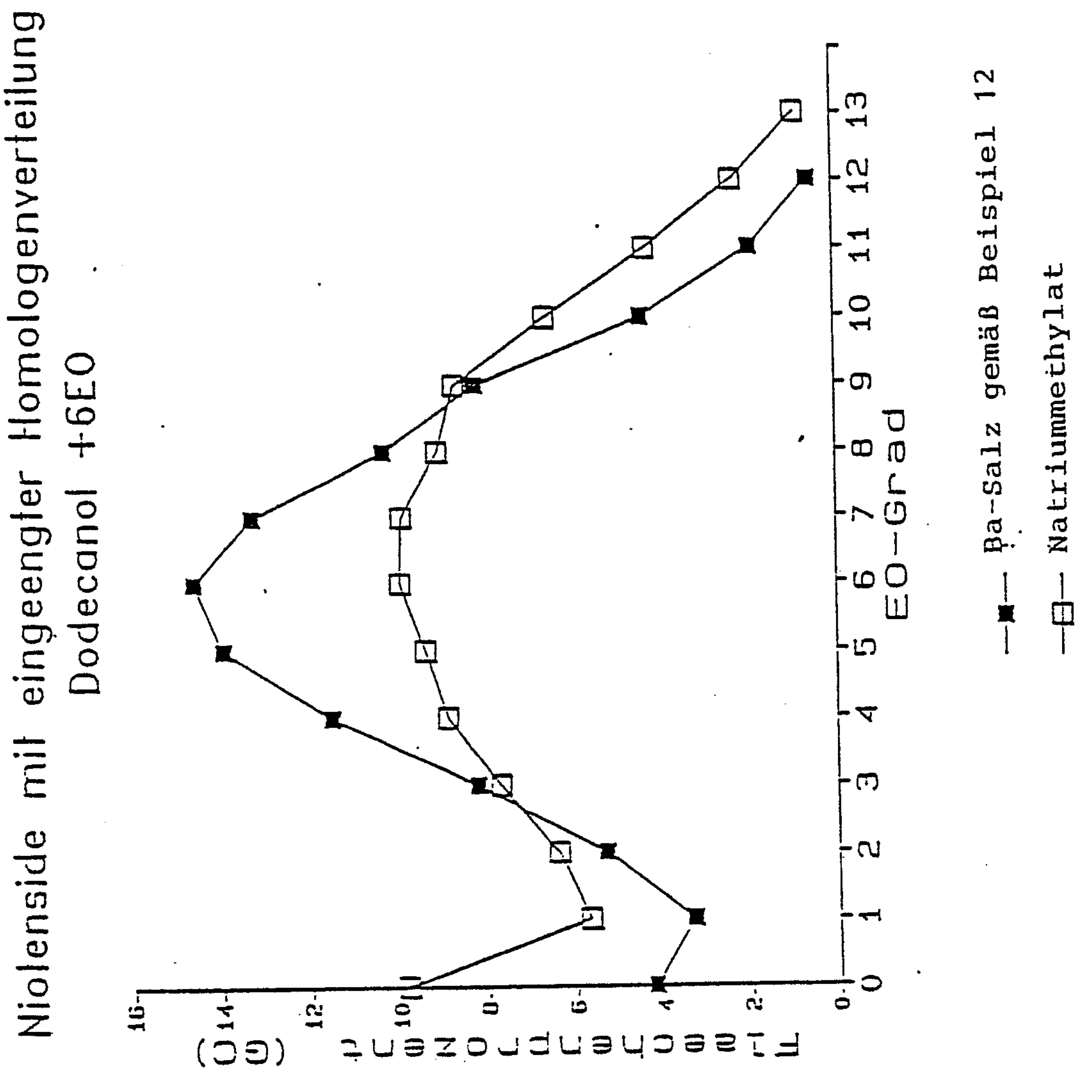


D 7464 EP